Europäisches Patentamt

European Patent Office

Office européen des brevets

(19)

(11) Veröffentlichungsnummer: **0 224 780**
**B1**

(12) **EUROPÄISCHE PATENTSCHRIFT**

(45) Veröffentlichungstag der Patentschrift:
28.02.90

(51) Int. Cl.⁴: **A61M 3/02**

(21) Anmeldenummer: 86115881.4

(22) Anmeldetag: 15.11.86

(54) **Vorrichtung zum Spülen des Darmes.**

(30) Priorität: 30.11.85 DE 8533814 U

(43) Veröffentlichungstag der Anmeldung:
10.06.87 Patentblatt 87/24

(45) Bekanntmachung des Hinweises auf die Patenterteilung:
28.02.90 Patentblatt 90/9

(84) Benannte Vertragsstaaten:
AT BE CH DE FR GB IT LI NL SE

(56) Entgegenhaltungen:
DE-A- 2 101 284

(73) Patentinhaber: **Beiersdorf Aktiengesellschaft,
Unnastrasse 48, D-2000 Hamburg 20(DE)**

(72) Erfinder: **Heimerl, Albert, Dr., Volksdorfer Weg 7b,
D-2071 Ammersbek(DE)**
Erfinder: **Hofeditz, Wolfgang, Dr., Vogt-Wells-Kamp 14,
D-2000 Hamburg 54(DE)**
Erfinder: **Brammer, Hans-Adolf, Taubenweg 1a,
D-2110 Buchholz(DE)**

## Beschreibung

Die Erfindung betrifft eine Vorrichtung zum Spülen des Darmes, die intraoperativ eingesetzt werden kann.

Eine Behinderung der Darmpassage, ein sogenannter Darmverschluß oder Ileus, kann durch funktionelle Störungen der Darmmotilität ohne anatomische Hindernisse oder durch mechanische Störungen verursacht werden.

Die Durchgängigkeit des Darmes kann bei mechanischen Verschlüssen nur auf operativem Wege wieder hergestellt werden. Beim Dickdarm-Ileus, häufig verursacht durch ein stenosierendes Karzinom, geht dem akuten Geschehen meist eine mehrtägige Verstopfung voraus. Mit zunehmendem Aufstau des Darminhaltes im gesamten Dickdarm und auch im Dünndarm entwickeln die Patienten dann Krankheitssymptome, die unter Bedingungen minimalster Vorbereitung notfallmäßig einen chirurgischen Eingriff erforderlich machen. Eine vorherige, sonst üblicherweise vor der Darmoperation durchgeführte mehrtägige Reinigung und Entlastung des Verdauungstraktes, um bei der Operation den Austritt von Keimen aus dem hochbesiedelten Darmlumen in die Bauchhöhle so gering wie möglich zu halten, ist bei derartigen Notsituationen nicht möglich.

Unter den Bedingungen der notfallmäßigen Resektion tumortragender oder anderweitig stenosierter Darmabschnitte versuchte man bisher in Fällen, bei denen keine entlastende Darmfistel angelegt wurde, mit ins Darmlumen eingeschobenen Intestinalsonden den Inhalt abzusaugen. Mit diser Methode ist jedoch nur eine unvollkommene Reinigung des Darmes möglich. Auch bisherige Versuche mit einer intraoperativen Spülung von oral nach aboral haben wegen der Wegführungsschwierigkeiten der austretenden Spülflüssigkeit aus dem Gefahrenbereich des Abdomens keine Vorteile gezeigt.

Der Erfindung liegt die Aufgabe zugrunde, mit einem bei unterschiedlichsten Darmdurchmessern verwendbaren Ableitungssystem die intraoperative Spülung des vor der Stenose liegenden Darmabschnittes zu ermöglichen, wobei jegliche Verschmutzung des Operationsfeldes auf der Seite der Ableitungsvorrichtung mit den bekannten, für den Patienten lebensbedrohenden Folgen, vermieden wird. Das System sollte außerdem den Richtlinien der Abfallentsorgung (gemäß Bundesgesundheitsblatt, Nr. 9 [1985]) entsprechen.

Die Aufgabe wird erfindungsgemäß gelöst durch eine Vorrichtung zum Spülen des Darmes, die intraoperativ verwendbar ist, mit einer Spülsonde zum Einleiten der Spülflüssigkeit und einer Ableitungsvorrichtung, die dadurch gekennzeichnet ist, daß die Ableitungsvorrichtung aus einem konischen Adapter mit keimdicht angefügtem Schlauch und Fixierungsmitteln zum Befestigen des Darmes am Adapter besteht.

Bei der Benutzung der Vorrichtung wird der Adapter mit dem angefügten Ableitungsschlauch in die orale Manschette des vor dem Engpaß liegenden Darmabschnittes eingeführt und mit Fixierungsbändern oder dergleichen, die außen um den Darmabschnitt festgezogen werden, wasserdicht befestigt. Über die Appendix oder eine weiter oralwärts angelegte Darminzision wird ein Spülkatheter (Spülsonde) in den Darm eingeführt und Spülflüssigkeit eingeleitet. Die Entsorgung der mit der Spülflüssigkeit vermischten Darmexkremente erfolgt in ein neben dem Operationstisch stehendes Auffanggefäß.

Der Adapter, der vorteilhafterweise aus einem physiologisch verträglichen Kunststoff im Spritzgußverfahren hergestellt worden ist, ist konisch ausgebildet und weist vorzugsweise ein oder mehrere Sollschnittstellen in Form von Schwächungslinien auf, um vor dem Einschieben unterschiedlichen Darmdurchmessern angepaßt werden zu können. Seine Öffnungen weisen vorzugsweise einen Durchmesser von 25 mm am einen Ende und 50 mm am anderen Ende auf, wobei der Querschnitt kreisförmig oder elliptisch ausgeformt sein kann. Die Wandstärke beträgt etwa 2 mm. Auf der Außenseite des Adapter befinden sich vorteilhafterweise eine oder mehrere versteifende Rippen mit einer Dicke von etwa 2 mm, die gegen das körperseitige (verjüngende) Ende des Adapters hin abgeschrägt sein können.

An der Seite des Adapters mit dem größeren Durchmesser ist der ableitende Schlauch, der vorzugsweise als Folienschlauch ausgebildet ist, befestigt. Die Befestigung wird in der Regel durch Kleben oder Verschweißen vorgenommen. Der Ableitungsschlauch mündet in einen Abfallauffangbehälter, der aus hygienischen Gründen möglichst dicht befestigt sein sollte. Ableitungsschlauch und Auffangbehälter können zu diesem Zweck einstückig ausgebildet sein, wobei der Auffangbehälter aus einem angeformten bzw. angeschweißten Folienbeutel bestehen kann. Auf diese Weise ist eine keimdicht zusammenhängende Ableitungs- und Auffangvorrichtung geschaffen.

Um auch auf der Seite der in den Darm eingeführten Spülvorrichtung (Katheter) eine Kontamination des Operationsfeldes zu verhindern, ist erfindungsgemäß ein passender Folienschlauch vorgesehen, der über die Sonde und das betreffende Darmstück, in das sie eingeführt ist, gezogen wird. Beim Herausziehen dient dieser schmiegsame Folienschlauch zur Abdeckung und zum Schutz des verschmutzten Katheters.

Als Fixierungsmittel zum Befestigen des Darmes auf dem Adapter können Bänder verwendet werden, die jeweils zwischen zwei versteifenden Rippen am Adapter angelegt werden, oder besser die aus der Packmittelindustrie bekannten Schnellverschlußbänder aus Kunststoff, wie sie beispielsweise zum Bündeln verwendet werden. Mit diesen läßt sich der Darm wasserdicht und rutschfest auf dem Adapter befestigen. Die Gestalt der Fixierungsbänder soll dabei so ausgelegt und diese so angelegt werden, daß der Darm gegen den ablaufenden Schenkel der Adapterrippen abgesperrt wird und somit die sichere Ableitung der angespülten Exkremente möglich ist.

Eine besonders vorteilhafte Ausführungsform einer derartigen Verschlußvorrichtung ist in Figur 2 dargestellt. Beim Anlegen des Bandes wird dieses zum Kreis geschlossen, wobei die Zacken a in die entsprechenden Vertiefungen b der U-förmigen Hinterschneidungszone c eingreifen. Die Hinterschneidungszone c ist dabei vorzugsweise leicht gekrümmt, um sich an die runde Kontur des Adapters besser anlegen zu können. Die Schlaufe d dient als Zuglasche.

Nachstehend wird die Vorrichtung anhand der Abbildungen beispielsweise erläutert:

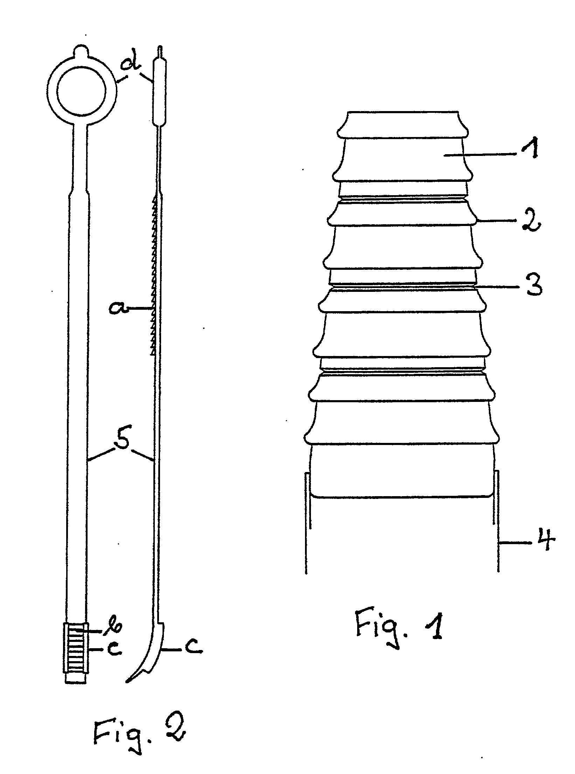

Figur 1 zeigt den konischen Adapter (1) im Querschnitt mit den versteifenden, abgeschrägten Rippen (2), den Schwächungslinien (Sollschnittstellen) (3) und dem angesetzten Ableitungsschlauch (4).

Figur 2 zeigt ein Schnellverschlußband (5) in der Aufsicht und im Querschnitt mit den Zacken a, den Vertiefungen b, der Hinterschneidungszone c und dem Zugring d.

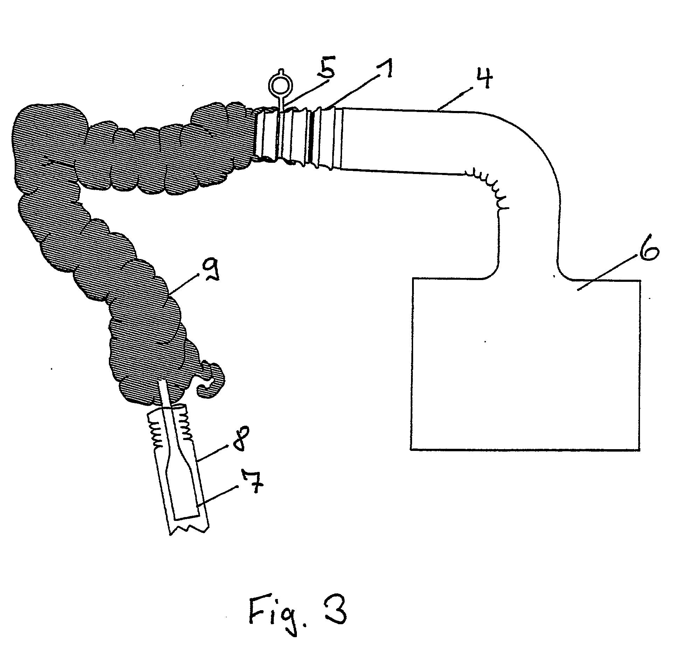

Figur 3 zeigt die erfindungsgemäße Vorrichtung in der Anwendung mit dem Adapter (1), dem Verschlußband (5), dem angefügten Ableitungsschlauch (4), dem Auffangbeutel (6), der Spülsonde (7), dem Folienschlauch (8) zum Abdecken der Spülsonde und einem Darmstück (9).

## Patentansprüche

1. Vorrichtung zum Spülen des Darmes, die intraoperativ verwendbar ist, mit einer Spülsonde (7) zum Einleiten der Spülflüssigkeit und einer Ableitungsvorrichtung, dadurch gekennzeichnet, daß die Ableitungsvorrichtung aus einem konischen Adapter (1) mit keimdicht angefügtem Ableitungsschlauch (4) und Fixierungsmitteln (5) zum Befestigen des Darmes am Adapter besteht.

2. Vorrichtung gemäß Anspruch 1, dadurch gekennzeichnet, daß der Adapter (1) eine oder mehrere Sollschnittstellen (3) aufweist.

3. Vorrichtung gemäß Anspruch 1, dadurch gekennzeichnet, daß der Adapter (1) auf der Außenseite eine oder mehrere versteifende Rippen (2) aufweist, die gegen das körperseitige Ende des Adapters hin abgeschrägt sind.

4. Vorrichtung gemäß Anspruch 1, dadurch gekennzeichnet, daß der an dem Adapter (1) befestigte Ableitungsschlauch (4) ein Folienschlauch ist.

5. Vorrichtung gemäß Anspruch 1, dadurch gekennzeichnet, daß an dem Ableitungsschlauch (4) ein Abfallauffangbehälter (6) befestigt ist.

6. Vorrichtung gemäß Anspruch 1, dadurch gekennzeichnet, daß der Ableitungsschlauch (4) und der Abfallauffangbehälter (6) einstückig ausgebildet sind.

7. Vorrichtung gemäß Anspruch 1, dadurch gekennzeichnet, daß das Fixierungsmittel (5) zum Befestigen des Darmes auf dem Adapter (1) als Schnellverschlußband ausgebildet ist.

8. Vorrichtung gemäß Anspruch 7, dadurch gekennzeichnet, daß das Schnellschlußband (5) eine Zackenreihe (a), entsprechende Vertiefungen (b) in der Hinterschneidungszone (c), die vorzugsweise leicht nach innen gekrümmt ausgebildet ist, und eine Zuglasche (d) aufweist.

9. Vorrichtung gemäß Anspruch 1, dadurch gekennzeichnet, daß die Spülsonde (7) von einem Folienschlauch (8) umgeben ist.

## Revendications

1. Dispositif pour laver l'intestin, qui peut être utilisé demanière intra-opératoire, comportant une sonde de lavage (7) pour introduire le liquide de lavage et un dispositif d'évacuation, caractérisé en ce que le dispositif d'évacuation est constitué d'un adaptateur conique (1) avec un flexible d'évacuation (4) qui y est raccordé d'une manière étanche aux germes et un moyen de fixation (5) pour la fixation de l'intestin à l'adaptateur.

2. Dispositif selon la revendication 1, caractérisé en ce que l'adaptateur (1) présente une ou plusieurs zones de coupe préférentielles (3).

3. Dispositif selon la revendication 1, caractérisé en ce que l'adaptateur (1) présente, sur sa face extérieure, une ou plusieurs nervures de renforcement (2) qui sont biseautées du côté de l'extrémité de l'adaptateur tournée vers le corps.

4. Dispositif selon la revendication 1, caractérisé en ce que le flexible d'évacuation (4) fixé à l'adaptateur (1) est un flexible en feuille mince.

5. Dispositif selon la revendication 1, caractérisé en ce qu'un récipient de collecte des déchets (6) est fixé au flexible d'évacuation (4).

6. Dispositif selon la revendication 1, caractérisé en ce que le flexible d'évacuation (4) et le récipient de collecte des déchets (6) sont réalisés d'une pièce.

7. Dispositif selon la revendication 1, caractérisé en ce que le moyen de fixation (5) pour fixer l'intestin à l'adaptateur (1) est constitué d'un collier à fermeture rapide.

8. Dispositif selon la revendication 7, caractérisé en ce que le collier à fermeture rapide (5) présente une série de dents (a), des creux correspondants (b) dans la zone de dépouille (c) qui présente, de préférence, une légère courbure vers l'intérieur et une patte de traction (d).

9. Dispositif selon la revendication 1, caractérisé en ce que la sonde de lavage (7) est entourée par un flexible en feuille mince (8).

## Claims

1. Device for lavage of the intestine, which device can be used intraoperatively, with a lavage probe (7) for introducing the lavage fluid and a drainage device, characterized in that the drainage device consists of a conical adaptor (1) with a drainage tube (4) joined on germproof and fixing means (5) for securing the intestine on the adaptor.

2. Device according to claim 1, characterized in that the adaptor (1) has one or more predetermined cutting points (3).

3. Device according to claim 1, characterized in that the adaptor (1) has on the outside one or more strengthening ribs (2) which are bevelled towards the body end of the adaptor.

4. Device according to claim 1, characterized in that the drainage tube (4) secured on the adaptor (1) is a film tube.

5. Device according to claim 1, characterized in that a waste-collecting container (6) is secured on the drainage tube (4).

6. Device according to claim 1, characterized in that the drainage tube (4) and the waste-collecting container (6) are designed in one piece.

7. Device according to claim 1, characterized in that the fixing means (5) for securing the intestine on the adaptor (1) is designed as a quick closure band.

8. Device according to claim 7, characterized in that the quick closure band (5) has a row of teeth (a), corresponding indentations (b) in the undercut zone (c), which is preferably designed curved slightly inwards, and a pull tab (d).

9. Device according to claim 1, characterized in that the lavage probe (7) is surrounded by a film tube (8).

Fig. 1

Fig. 2

Fig. 3